# EUROPEAN PATENT SPECIFICATION

(11) **EP 2 107 917 B1**
(45) Date of publication and mention of the grant of the patent: **12.08.2020**
(21) Application number: 08708107.1
(22) Date of filing: 23.01.2008
(51) Int. Cl.: A61M 25/00, A61M 5/158

(54) **INJECTION SITE FOR INJECTING MEDICATION**
EINSTICHSTELLE FÜR DIE INJEKTION EINES ARZNEIMITTELS
LIEU D'INJECTION POUR INJECTER UN MÉDICAMENT

(30) Priority: 02.02.2007 DK 200700179; 02.02.2007 US 899143 P; 02.02.2007 DK 200700185; 02.02.2007 US 899075 P; 02.02.2007 DK 200700191; 02.02.2007 US 899062 P
(43) Date of publication of application: 14.10.2009
(73) Proprietor: Unomedical A/S, 3460 Birkerød (DK)
(72) Inventor: MATHIASEN, Orla, 4180 Sorø (DK); THEANDER, Julie Grundtvig, 3650 Ølstykke (DK)
(74) Representative: Wilson Gunn
(86) International application number: PCT/EP2008/050742
(87) International publication number: WO 2008/092782

(56) References cited:
- WO-A-02/053220
- WO-A-03/075980
- WO-A-2006/015507

## Description

This invention relates to a device such as an injection site or a gateway. The device is placed partly subcutaneously in relation to the patient for as long as e.g. three days and replaces repeated injections by syringes or injection pens thereby reducing trauma to the patients' skin while it at the same time keep the injection place free of infections. Such a device is disclosed in the WO2006015507.

Injection sites and gateways as such are already known. In previous documents the use of a needle assembly comprising a gateway and a pen-type injector is disclosed and by such an assembly it is possible to provide subcutaneous or intravenous injections using a blunt tipped needle. It is not indicated in the documents how the gateway is inserted. It would not be possible to use even a relatively short, sharp needle for injection in this gateway as the risk of penetrating the side of the soft cannula with a hypodermic needle would be considerable, as the steering or piloting of the needle when penetrating the septum is small and at the same time the hard case housing is very short.

Also other types of gateways are known, e.g. gateways comprising an elongated housing having an internal passageway extending from one end of the housing to the opposite end in the longitudinal sense. A cannula tube will be connected to the housing and extends from the distal end of the passageway. The cannula tube is connected to the housing by means of a bushing and immediately adjacent to the proximal end of the cannula is a self-sealing silicon membrane. The membrane is in the form of a plug engaging the rear end of the bushing. In this way there is only a minimum of dead space i.e. internal volume in the passageway of the housing. This gateway has a rather long hard case housing which reduces the risk of penetrating the cannula with a sharp needle, but the gateway is also intended to be inserted manually in a very low angle. After insertion the gateway is placed almost parallel to the patients' skin and this parallel position can make it difficult for the patients themselves to inject medical substances through the gateway.

It is an aim of the present invention to provide a gateway which is easy for the patient to place and to use for self-administration of drugs or other medicaments. Also it is an aim that the gateway after placement onto the patients' skin is noticed as little as possible by the patient when the patient is not actually injecting medication.

In US 2007/0129688 is shown an insertion head for a medical or pharmaceutical uses including a housing with a side which can be positioned and fastened to the skin surface of a patient and a penetrating device with a tip. The penetrating device can be carried by the housing in a protected orientation in which the housing covers the tip and in a penetrating orientation in which the tip protrudes in the insertion angle. The penetrating orientation is angled relative to the protected orientation and the penetrating device including the access part which it penetrates can be pivoted from one position to the other. This device is intended for use together with a connector (18) connecting the infusion part to an infusate supply line (19) and the flow path through the cannula and through the connecting structure (6) always form a straight line. It would not be possible to use even a relatively short, sharp needle for injection in this device.

### Summary of the invention

The present invention relates to a device for supplying medication as further disclosed in claim 1, comprising a base part having means for securing the base part to the skin of a patient, a cannula unreleasably connected to the base part and a through-going opening providing access of fluid to the cannula, where the through-going opening has an access opening and provides fluid access to the cannula at an exit opening. The access opening and at least a part of the through-going opening are located in a gate which gate is permanently attached to the base part and is movably mounted in relation to the base part. This means that the gate after the cannula has been inserted has at least one position in which position there is an angle a between a longitudinal central axis (l₂) of the gate and the longitudinal central axis of the cannula and that the angle a is different from 0° ± n•180°, where n is an integer., i.e. the flow path through the gate and through the cannula does not always form a straight line.

Normally α ∈ [90°, 170°] but it would be possible to pivot the gate in the direction of the inserted cannula which would cause α to be smaller than 90°, and then α ∈ [10°, 90°[. Preferably α ∈ [30°, 150°].The gate thereby provides a mouthpiece or a hopper which can be moved in relation to the base part after the base part has been fastened to the patient's skin making it easier to inject medication i.e. the gate provides a position for use of a pointed needle. That the gate is permanently attached to the base part means that it cannot be removed from the base part within ordinary use of the equipment, removing the gate from the device would involve violation of the equipment. The possibility of moving the trough-going opening and thereby moving the access opening of the through-going opening provides the user with the possibility of choosing the general position or angle from where the user wants to inject the medication to the patient. If the user is the patient himself it opens up for the possibility that the device can be placed at a position on the patient's skin where access for injection is not normally easy.

According to an embodiment the cannula or a fluid tight continuation of the cannula is flexible. When the cannula or a part of the gate is flexible - depending on where the limit between the two parts is defined - it is possible to move the gate after the cannula has been inserted into the patient.

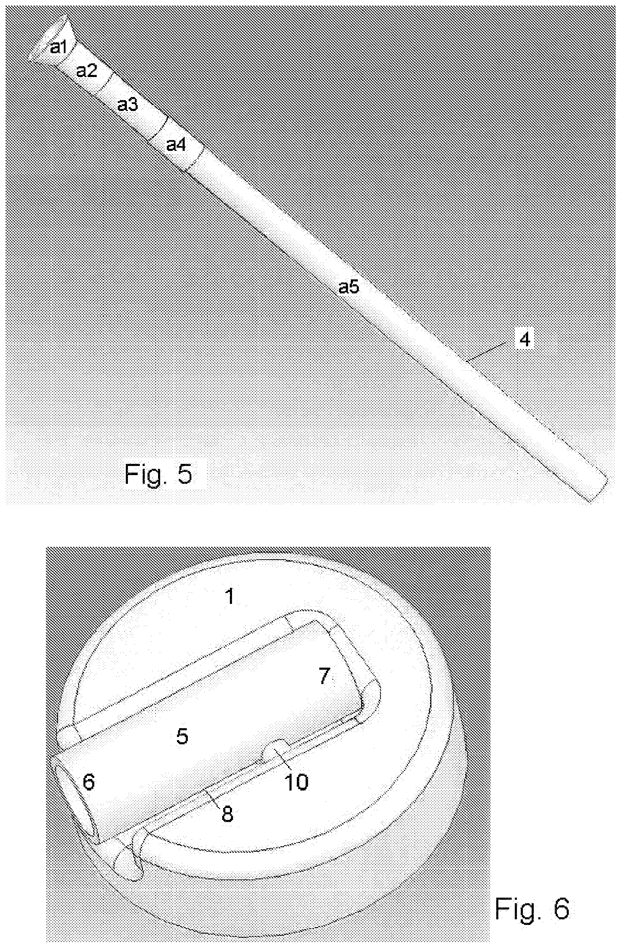

According to an embodiment the cannula is constructed of a soft material and is provided with a flexible joint close to the exit end of the gate. Such an embodiment is shown in fig. 5.

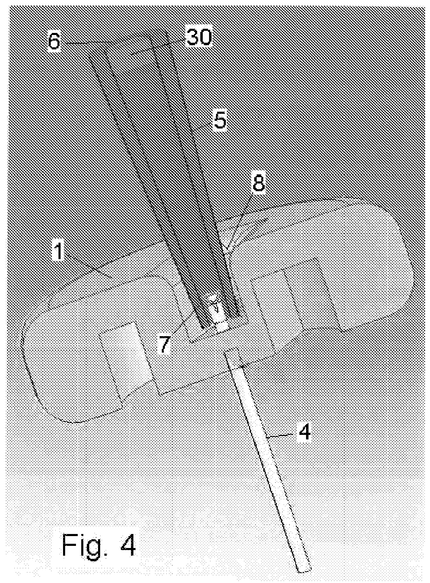

According to an embodiment the through-going opening is provided with means protecting the cannula from the access of micro organisms. These means can be an ordinary septum as e.g. shown in figure 4 but any means not disturbing the functioning of the device will be acceptable.

According to an embodiment the gate and the cannula are fixed in relation to each other. This means that the gate 5 and the cannula do not move in relation to each other, if one of them moves the other moves as well i.e. they performs as one unit and might be produced as one unit.

According to an embodiment the through-going opening of the gate and the through-going opening of the cannula are in direct continuation of each other i.e. the two units are joined to each other providing a fluid tight flow through the united parts.

According to an embodiment the through-going opening of the gate and the through-going opening of the cannula have coincident axis (l₂). In this embodiment the central axis 12 is a straight line through both the gate 5 and the cannula 4.

According to an embodiment the access opening of the through-going opening of the gate will in at least one position be positioned close to the patient's skin and in at least one other position the access opening is pointing away from the patient's skin. When the access opening 6 of the through-going opening can be placed close to the skin of the patient, this means that the gate 5 is placed in a position in a plane at least approximately parallel to the patient's skin. The device according to the invention is intended for fastening to the patient's skin for a longer period e.g. up to three days and the device will normally be small and flat in order for the patient not to be adversely affected by the device e.g. by the device being caught in anything or just being visible. Therefore it is an advantage that the movable gate 5 according to the invention can be placed in a position close to the skin while not in active use, i.e. while not being used for injecting medication through the access opening of the gate.

According to an embodiment the gate is tubular and can pivot around an axis l₁ which axis l₁ is positioned in a direction perpendicular to the plane comprising the longitudinal axis l₂ of the tubular gate. That the gate is tubular means that it has an inner opening with a certain length through which opening fluid flows.

According to an embodiment the gate can move between an angle α1 and an angle α2 by pivoting the gate around the axis l₁, where α1 ≥ 0 and α2 ≤ 90. According to one embodiment of the device the tubular gate is pivotally mounted around an axis which is stationary in relation to the base part 1. This construction is simple and non-expensive to produce as the two cooperating parts - the base part and the gate - can be moulded individually in e.g. a hard plastic. Actually the gate 5 can be positioned in an angle α1< 0 if the axis l₁ is positioned adequately high and the opening 8 in the base part 1 allows for access opening of the gate 5 to be positioned low enough. Normally the access opening 6 of the gate will move along a straight line when employing this embodiment.

According to an embodiment the axis l₁ is positioned below the outer surface of the base part in connection with the exit end of the gate. "In connection with" means that the axis is placed within half the length of the gate closest to the exit end 7, or even within a third of the length of the gate closest to the exit end 7 of the gate 5. When the axis l₁ is positioned below the upper surface of the base part 1 the height of the device is reduced.

According to an embodiment either the gate or the base part is provided with rotational-symmetrical protruding parts and the other part is provided with corresponding cavities or openings positioned at the rotation axis l₁ of the gate. According to this embodiment the gate 5 is fastened to the base part by a hinge joint i.e. a joint which permits motion in only one plan. The embodiment provides an unreleasable fastening of the gate 5 to the base part 1 but allows the gate 5 to move in relation to the base part 1.

According to an embodiment the gate is unreleasably mounted in relation to the base part. That the gate 5 is unreleasably mounted in relation to the base part means that it is not possible for the user or the patient to remove the gate from the base part 1 without violating the device.

According to an embodiment the base part comprises an opening in which the cannula can be placed in extension of the gate. In this embodiment the mounting pad 2 - as far as the device is provided with a mounting pad 2 - should of cause be provided with a corresponding opening in order to allow the cannula 4 to pass into the opening of the base part 1.

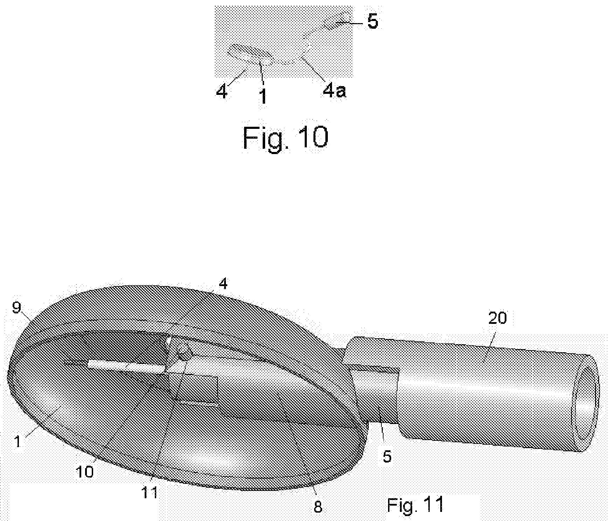

According to an embodiment the fluid tight continuation of the cannula is constructed of a soft material and forming a conduit connecting the gate with the cannula. This embodiment is shown in fig. 10.

According to an embodiment the gate can be moved between two end positions, where the gate in the first position is placed along a plane parallel to the patients skin and the gate in the second position is positioned with the exit opening directly secured to the cannula and the access opening pointing away from the plane parallel to the patients skin in a predefined angle α. According to an embodiment the predefined angle α belongs to the interval 45 degrees ≤ α ≤ 90 degrees. According to an embodiment the predefined angle α is approximately 90 degrees.

According to an embodiment the base part is less than 0.010 m in height where it is highest and less than 0.030 m wide in the dimension where it is widest. According to an embodiment the base part is less than 0.006 m in height where it is highest and less than 0.020 m wide in the dimension where it is widest.

The invention also relates to a kit comprising both a device according to claim 1-9 and an injection device, wherein the access opening and the cannula of the base part are placed such that an injection needle can be aligned with both. The position or positions where the injection needle 9 is aligned with the cannula 4 defines the positions before use, i.e. before insertion, where the pointy insertion needle 9 and the cannula 4 are either in a protected pre-insertion position where the surroundings are protected from the insertion needle or in a position where the user has prepared the device for insertion and has decided on an insertion angle from which angle the cannula 4 of the device can be injected subcutaneously into the patient. The straight line formed by the injection needle and the cannula 4 in relation to the lower or distal surface of the base part 1 defines the insertion angle for the user. According to the present invention the device can be fitted at any angle, according to the device of claim 1 there are no restriction on the insertion angle, it could be anywhere between 45 and 90 degrees.

According to an embodiment of the kit, the injection device can be placed in a protected position where the insertion needle is positioned inside the base part.

According to an embodiment of the kit the mounting pad is provided with an opening through which opening the injection needle can pass. When the mounting pad 2 is provided with an opening 2a through which it is possible to pivot the injection needle 9 aligned with the cannula 4, it is possible to use the base part 1 as a protecting shield for the insertion needle 9 before use. The insertion needle 9 together with the cannula 4 and the gate 5 can be pivoted from a position practically parallel to lower/proximal surface of the base part 1 where the insertion needle 9 and the cannula 4 are hidden inside the base part 1 to the insertion position which could be e.g. 45 degrees in relation to the lower side of the base part 1 or any other angle the user might prefer.

According to an embodiment of the kit the injection device can be placed in any angle ≤ 90 degrees in relation to the lower/distal surface of the base part.

Further the kit can be delivered to the user in a packing under sterilised conditions.

Embodiments of the invention will now be described with reference to the figures in which:
Figure 1a, 1b and 1c shows one embodiment from three different views: fig. 1a shows the device seen from above; fig. 1b shows the device seen from the side having the gate in an upright position, and fig. 1c shows a cut through the device at the line c-c shown in fig. 1a.
Figure 2 shows the same embodiment as fig. 1a-1c where the gate is in a position parallel to the patient's skin.
Figures 3 shows an embodiment of the device where the gate has the form of a truncated cone.
Figure 4 shows a cut through the same embodiment as shown in fig. 3.
Figure 5 shows an embodiment of a flexible cannula which can be used in the device.
Figure shows a third embodiment of a device according to the invention in a position where the gate is parallel to the patients skin.
Figure 7 shows a device according to the invention in a position where it is ready for injection of the cannula in an angle smaller than 90 degrees.
Figure 8a and 8b show an embodiment where the axis, around which the gate is rotatably mounted, is positioned below the upper surface of the base part.
Figure 9 shows another embodiment where an insertion needle is positioned inside the cannula when the gate is in a first position intended for pre-use and in a second position intended for insertion of the device.
Figure 10 shows an embodiment of a device according to the invention where the gate is mounted to the base part via a flexible extension of the cannula.
Figure 11 shows an embodiment of the device seen from below without the adhesive pad and the release liner in a before-use position.
Figure 12 shows the same embodiment seen from the side in a ready-to-insert position.
Figure 13 shows a cut-through view of the same embodiment as shown in fig. 12 and 13 in the same position as in fig. 12.
Figure 14 shows a device according to the invention which is to be placed with a manual inserter.
Figure 15 shows an integrated adaptor for a medication pen provided with a needle.
Figure 16a-c show an integrated adaptor for a needleless medication pen.

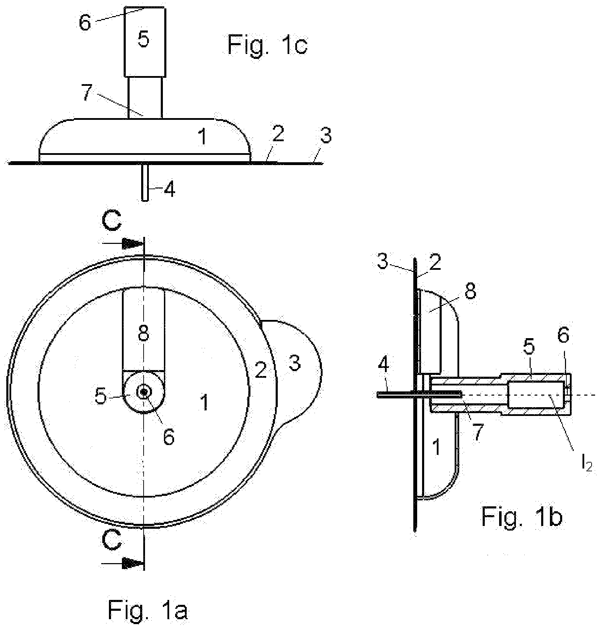

Fig. 1a, 1b and 1c show a device according to the invention seen respectively from above, from the side and from a cut through the line indicated in fig. la. The device comprises a base part 1 with a smooth distal surface, which distal surface is the surface turned away from the patient after the device has been inserted. A mounting pad 2 having an adhesive surface is placed at the proximal side of the base part 1, i.e. the side of the device turned toward the patient during use. The mounting pad 2 is covered with a release liner 3 which release liner 3 protects the adhesive surface of the mounting pad 2 before insertion of the device. The base part 1 has a through-going opening in which a cannula 4 is located; the cannula 4 extends from the proximal side of the base part 1. At the distal end the cannula 4 is embedded in a gate 5 having an access opening 6 and an exit opening 7, in fig. 1a, 1b and 1c the gate 5 is in a second position where the access opening 6 of the gate 5 is pointing away from the plane parallel to the patients skin in an angle of 90 degrees. The gate 5 is normally provided with a not shown restriction such as a septum which prevents access of micro organisms. A septum limits access to the through going opening of the gate 5 as an opening created in the septum by a needle will close after removal of the needle due to the characteristics for the material chosen for the septum . It is necessary to use a relatively hard needle to penetrate the septum, but the needle does not need to be pointy. Relatively hard means that the needle has to be harder than the material of the septum, the needle need not be made of steel; it could be made of e.g. hard plastic. In EP 1191964 it is described how to produce such a needle.

The base part 1 is provided with a cavity 8 which in this embodiment reaches from the center of the base part 1 to the periphery and is wide enough to receive the gate 5. The gate 5 can be placed inside the cavity 8 after the not shown insertion needle has been removed from the cannula 4, if it is possible to bend the cannula 4. The gate 5 is in this embodiment fastened to the base part 1 with a hinge joint and when the gate 5 is moved, the gate 5 is rotated around an axis I₁ placed below the upper surface of the base part 1. The gate 5 can be pivoted from the upright second position to this first position along a plane parallel to the patient's skin. In order for the gate 5 of this embodiment to get from the second to the first position it is necessary for the cannula 4 to be able to flex in an angle of approximately 90 degrees. When the gate 5 is brought to this position after insertion of the device the risk of the gate catching anything while attached to the patient is greatly reduced compared to the situation where the gate 5 is in an upright position. If the end of the gate 5 comprising the access opening 6 was approximately at level of the outer surface of the base part 1, it would not matter whether the gate 5 remained in the upright position or was returned to the position close to the patient's skin.

The gate 5 can also be joined to the base part 1 with a ball joint, it will then be possible to move the gate in more than one plane.

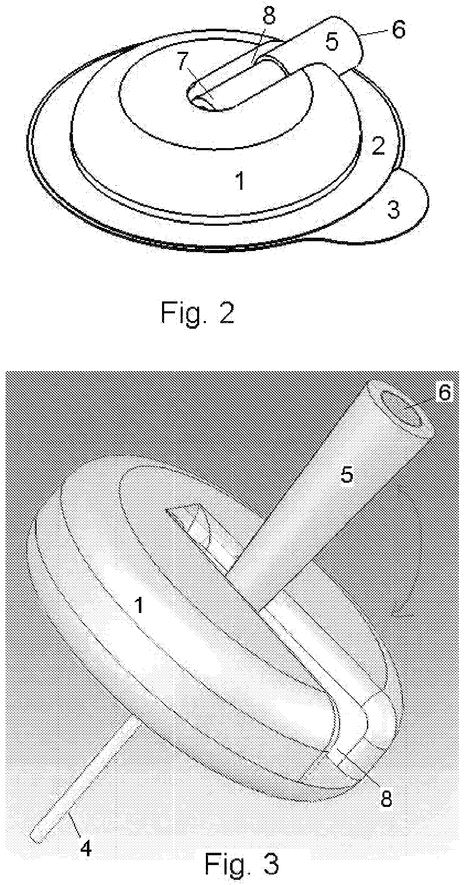

Fig. 3 and 4 show another embodiment of a device according to the invention. In fig. 3 this embodiment of the device is shown in a position where the gate 5 is coaxial with the cannula 4 and this position is the position in which the device would be placed subcutaneously if the user desires an insertion angle of 90 degrees. The outer surface of the gate 5 of this embodiment is shaped as a truncated cone. Fig. 4 shows a cut through the same embodiment as shown in fig. 3 in the same position. From this view it is possible to see that a septum 30 is placed at the access end 6 of the trough going opening of the gate 5 and the distal end of the cannula 4 is embedded in relation to the exit opening of the gate 5. A fluid tight connection is provided between the gate 5 and the cannula 4.

Fig. 5 shows an embodiment of a cannula 4 provided with a flexible joint. The flexible joint is provided by constructing the cannula 4 of different materials having varying flexibility. The cannula 4 shown in fig. 5 is divided into five different areas, a1-a5. The area a1 has the form of a truncated cone and is normally embedded in the base part 1; the truncated cone can be made either of a rigid or of a soft and flexible material as the small dimension assures that whichever material is chosen, the cannula 4 can not be pulled out of the base part 1. The second area a2 is made of a relatively rigid material which is not able to bend under conditions represented during normal use. The third area a3 is made of a soft and flexible material which material will be easily bend under normal use-conditions. The fourth area a4 is also made of a relatively rigid material as described for the third area a3. The fifth area a5 is the part of the cannula 4 which is inserted subcutaneously into the patient, this material could be either a soft and flexible material or it could be a rigid material such as steel.

Fig. 6 shows a third embodiment where the gate 5 is constructed of a tubular part having constant diameter over the whole length. The gate 5 is shown in a position where it would be placed either after insertion of the cannula 4 or before insertion of the cannula 4. In the first case the cannula 4 is inserted subcutaneously into the patient's skin and bend (not shown) in an angle corresponding to the insertion angle in order to maintain the fluid tight connection with the gate 5. In the second case the cannula 4 would be placed coaxially in extension of the exit end 7 of the gate 5 inside the base part 1.

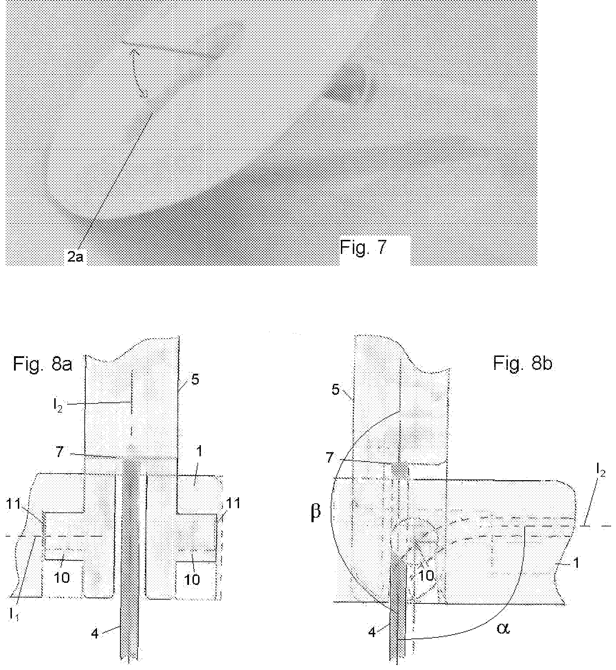

Fig. 7 shows an embodiment of the device seen from below. From this position it is possible to see the opening 2a through which the cannula 4 can pass when shifting from one position to another. In fig. 7 the gate 5 and the cannula 4 are positioned coaxially in an angle of approximately 45 degrees in relation to the plane coinciding with the proximal surface of the base part 1. A manual insertion device is connected to the device providing a kit. The insertion needle 9 of the insertion device is placed along the common axis of the gate 5 and cannula 4 and extends beyond the end of the cannula 4. Before insertion the insertion needle 9 would be placed inside the base part 1 when the user intends to insert the cannula 4, he will pivot the insertion needle 9 into the wanted angle and then insert the insertion needle 9 together with the cannula 4 subcutaneously. After insertion the user will remove the insertion needle 9, and if the cannula 4 is of a soft or at least bendable type he can push the gate 5 into a position parallel to the patient's skin.

Fig. 8a and 8b show an embodiment of the mounting of the gate 5 in the base part 1.

Fig. 8a shows the fastening of the gate 5 from the side perpendicular to the axis l₁ around which the gate 5 pivot. Fig. 8b shows the fastening of the gate 5 from the side along the axis l₁ around which the gate 5 pivot. In this embodiment the gate 5 is connected unreleasably i.e. permanently to the base part 1 with a hinge joint. The hinge joint is constructed of two protruding parts 10 protruding from opposite sides of the tubular gate 5. The protruding parts fit into corresponding openings 11 in the base part 1 and make it possible for the gate 5 to pivot from an upright position (shown in figs. 8a and 8b) to a position parallel to the proximal surface of the base part 1. For this embodiment the upright position where the angle β between the longitudinal axis of the gate 5 and the longitudinal axis l_{c} of the cannula is 0° or 180°, can be both a position for inserting the cannula and a position where it is possible to supply medication to the patient via the mounting gate 5. The protruding parts 10 define the axis l₁ around which the gate 5 moves.

In fig. 8b the position of the bend cannula 4 is indicated with dotted lines, this position of the cannula 4 is achieved when the gate 5 is brought into the position parallel to the patient's skin. In this position the angle α between the longitudinal axis of the gate 5 and the longitudinal axis l_{c} of the cannula is approximately 90°, and in this position the mounting gate might be hidden and it is not possible or at least not easy to supply medication to the patient via the mounting gate 5.

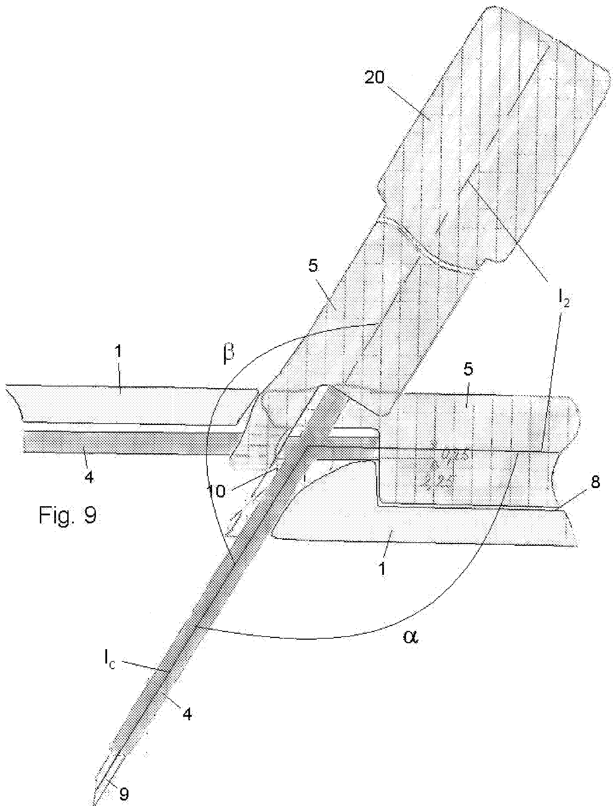

Fig. 9 shows an embodiment of the device together with an insertion device 20 in a view along the axis around which the gate 5 can pivot. In fig. 10 the gate 5 is shown in two positions, an upright position where the gate 5 and the cannula 4 are coaxially positioned in an angle of approximately 60 degrees in relation to the proximal surface of the base part 1, and a low position where the gate 5 and the cannula 4 are also coaxially positioned now in an angle of approximately 0 degrees in relation to the proximal surface of the base part 1 In both positions the angle β is 0° or 180°. The gate 5 and the cannula 4 will be in the low position before insertion of the cannula 4 and the simultaneously placement of the base part 1 on the skin of the patient, and they will be in the upright position at the moment where the user intends to insert the insertion needle 9 and the cannula 4 which is firmly connected to the insertion needle 9. After insertion of the cannula 4 the gate can be brought to the position close to the patients skin and the angle α between the longitudinal axis of the gate 5 and the longitudinal axis l_{c} of the cannula could then be approximately 120°,

Fig. 10 shows a fourth embodiment which is provided with a fluid tight continuation 4a of the cannula 4. The fluid tight connection 4a consist of an elongated tubing made of a soft and bendable material, the tubing connects the gate 5 with the cannula 4, the cannula 4 and the connection 4a are joined fluid tight together inside, i.e. behind the outer surfaces of the base part 1. This embodiment makes it possible to design the base part 1 as a very thin unit which will cause the patient very little discomfort as it is kept close to the patient's skin. The device according to this embodiment can further be provided with guiding means for the elongated tubing 4a i.e. the tubing can be coiled at least partly around the device. The device can also be provided with means such as an adhesive or a magnet for fastening of the gate 5 e.g. to the base part 1 or to the mounting pad 2.

Fig. 11 shows a fifth embodiment of the device combined with a manual insertion device 20 in a position used before insertion of the cannula 4. The insertion needle 9, the cannula 4 and the gate 5 are coaxially positioned along a plane parallel to the proximal surface of the base part 1. The cannula 4 which is closely adjoined to the insertion needle 9 is positioned in the hollow inside of the base part 1. This view shows the protruding parts 10 around which the gate 5 is pivoted together with the openings 11 in the base part 1. When the kit is prepared for insertion of the device, the gate 5 is lifted to an upright position in an angle preferred by the user. Then the kit is pushed toward the patient's skin in conformity with the chosen insertion angle. After insertion the insertion device including the insertion needle 9 is removed and it will now be possible to return the gate 5 to the position where it lies parallel with the patient's skin.

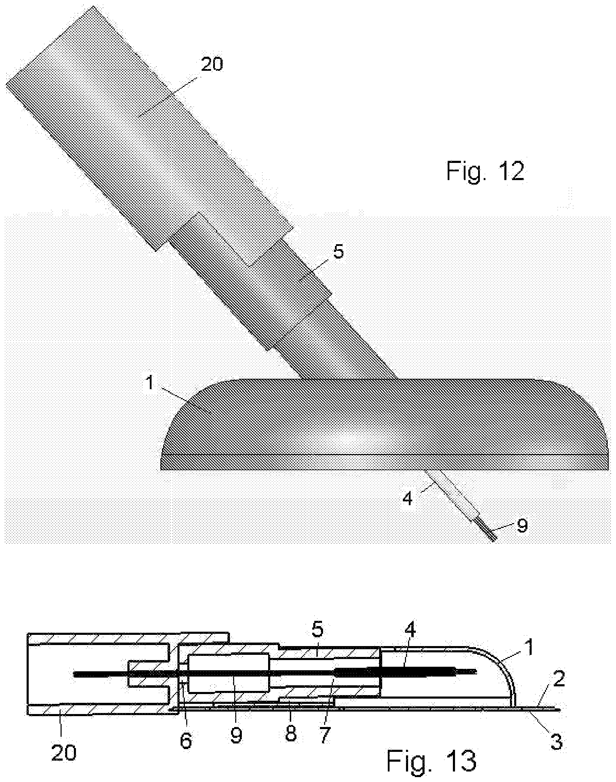

Fig. 12 shows the embodiment of fig. 12 in an upright position ready for insertion of the device.

Fig. 13 shows the same embodiment as fig. 12 and 13 in a cut-through view where the gate is in the position the same position as shown in fig. 12, i.e. the gate 5 lies approximately parallel to the patient's skin.

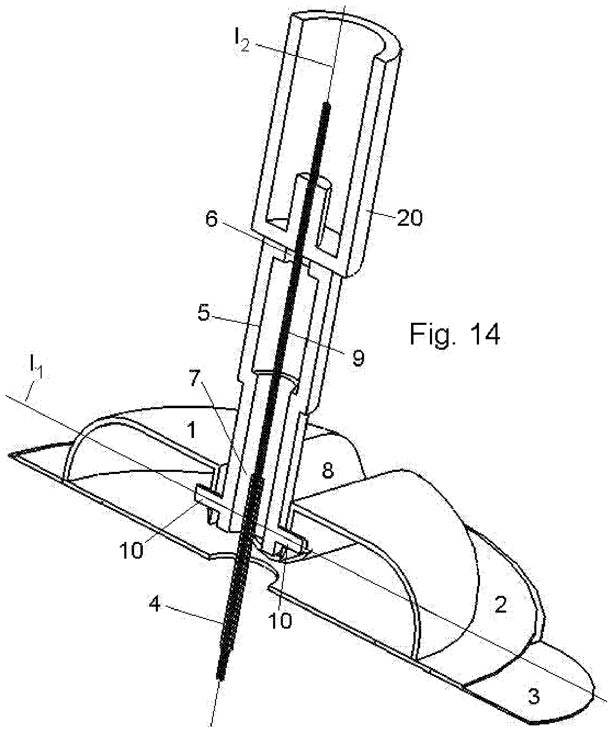

Fig. 14 shows a cut-through view of a device provided with a mounting pad 2 together with a manual insertion device 20. This view shows the axis l₁ around which the gate 5 pivot and it also shows the axis l₂ which is the common centre of the gate 5, the cannula 4 and the insertion device 20. In this embodiment the axis l₂ is coinciding with the insertion needle 9.

The insertion device 20 used in fig. 14 is a manual insertion device, and a manual insertion device basically just comprises a handle 20 and an insertion needle 9. The insertion is executed with a manual insertion device the user forces the insertion needle 9 through the skin of the patient and after insertion the insertion device is removed and the cannula 4 is left subcutaneously.

The insertion can also be executed with an automatic insertion device of the type illustrated in WO03/026728. When using an automatic device the user first places the insertion device against the skin of the patient and the user then activates the insertion device, after insertion the insertion device is removed together with the insertion needle. An automatic insertion device will normally assure that the base part 1 is inserted in a predefined angle and depth using a predefined force.

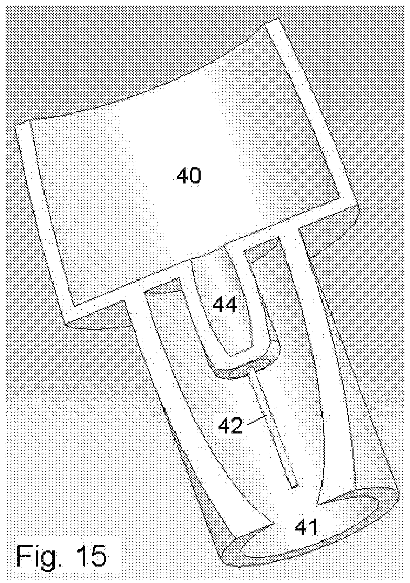

Fig. 15 shows an integrated adaptor which can be used together with a medication pen, e.g. a pen for delivering insulin. The adaptor has a receiving part 40 which in this embodiment is intended for receiving the outlet end of a medication pen provided with a needle. The needle from the medication pen will upon insertion of the head of the medication pen into the adaptor be placed in the needle space 44. A part of the needle space 44 can be provided with a septum or a similar plastic material in order to reduce the dead space in the needle space 44. Also such a plastic material could be used to keep the medication pen attached to the adaptor as the plastic material could be chosen such that it would squeeze firmly around the insertion needle of the medication pen. The adaptor also has a protecting shield 41 which prevent the insertion needle 42 from getting in contact with the surroundings. The insertion needle 42 is unreleasably fastened to the adaptor.

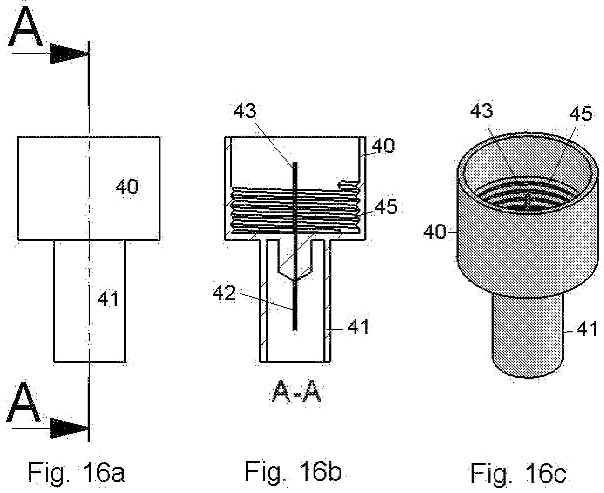

Figs. 16a-c also show an integrated adaptor which can be used together with a medication pen. Fig. 16a shows a side view of the device, fig. 16b shows a cut-through view along the line A-A and fig. 16c shows a spatial view from above. This adaptor can be used together with medication pens without an insertion needle. The adaptor has - similar to the embodiment of fig. 15 - a receiving part 40, a protective shield 41 and an insertion needle 42 which can be either blunt or sharp, this depend on whether the pen/adaptor combination is used to inject medication e.g. to a device having a soft septum or subcutaneously.

This embodiment of the adaptor is further provided with a needle 43 pointing into the receiving part 40 and this needle 43 would penetrate a protective layer covering the outlet from the medication pen. This protective layer prevents access of micro organisms to the medication reservoir inside the medication pen. Further this embodiment is provided with a screw thread 45 which is an alternative way of keeping the adaptor in the correct position in relation to the medication pen. Normally the integrated adaptor will be delivered together with the medication pen in a sterile packing to the user, the user will then have more than one possibility when choosing how to inject the medication.

## Claims

1. Device for supplying medication comprising a base part (1) having means (2) for securing the base part (1) to the skin of a patient, a cannula (4) unreleasably connected to the base part (1) and at least partially extending from the proximal side of the base part (1) and a through-going opening providing access of fluid to the cannula (4), where the through-going opening has an access opening (6) and an exit opening (7), the access opening (6) and at least a part of the through-going opening are located in a gate (5) permanently attached to the base part (1) and pivotable in relation to the base part (1) **characterized in that** the gate (5) after the cannula (4) has been inserted and the insertion needle has been removed, has at least one position in which position there is an angle α between a longitudinal central axis (l2) of the gate (5) and the longitudinal central axis of the cannula (4) and that the angle α is different from 0° and/or 180°.

2. Device according to claim 1, **characterized in that** the cannula (4) or a fluid tight continuation of the cannula (4) is flexible.

3. Device according to claim 2, **characterized in that** the cannula (4) is constructed of a soft material and is provided with a flexible joint close to the exit end (7) of the gate (5).

4. Device according to claims 1-3, **characterized in that** the through-going opening is provided with means (30) protecting the cannula (4) from the access of micro organisms.

5. Device according to any of claims 1-4, **characterized in that** the through-going opening of the gate (5) and the through-going opening of the cannula (4) have coincident axes (I₂).

6. Device according to claim 1, **characterized in that** the gate (5) is tubular and that the gate (5) can pivot around an axis l₁ which axis l₁ is positioned in a direction perpendicular to the plane comprising the longitudinal axis l₂ of the gate (5).

7. Device according to any of claims 1-6, **characterized in that** the axis l₁ is positioned below the outer surface of the base part (1) in connection with the exit end (7) of the gate (5).

8. Device according to claim 7, **characterized in that** either the gate (5) or the base part (1) is provided with rotational-symmetrical protruding parts (10) and the other part is provided with corresponding cavities or openings (11) positioned at the rotation axis l₁ of the gate (5).

9. Device according to claims 6-8, **characterized in that** the gate (5) is unreleasably mounted in relation to the base part (1).

10. Kit comprising a device according to claims 1-9 and an injection device (20), wherein the access opening (6) and the cannula (4) of the base part (1) are placed such that an injection needle (9) can be aligned with both.

11. Kit according to claim 10, **characterized in that** the injection device (20) can be placed in a protected position where the insertion needle (9) is positioned inside the base part (1).

12. Kit according to claim 11, **characterized in that** the mounting pad (2) is provided with an opening (2a) through which opening the injection needle (9) can pass.

13. Kit according to claims 10-12, **characterized in that** the injection device (20) can be placed in any angle ≤ 90 degrees in relation to the lower/distal surface of the base part (1).

14. Kit according to claims 10-13, **characterized in that** the kit is delivered to the user in a packing under sterilised conditions.

## Patentansprüche

1. Vorrichtung zum Zuführen von Medikamenten, umfassend ein Basisteil (1) mit Mitteln (2) zum Befestigen des Basisteils (1) auf der Haut eines Patienten, eine Kanüle (4), die unlösbar mit dem Basisteil (1) verbunden ist und sich zumindest teilweise von der proximalen Seite des Basisteils (1) aus erstreckt, und eine Durchgangsöffnung, die den Zugang von Flüssigkeit zu der Kanüle (4) ermöglicht, wobei die Durchgangsöffnung eine Zugangsöffnung (6) und eine Austrittsöffnung (7) aufweist, die Zugangsöffnung (6) und mindestens ein Teil der Durchgangsöffnung in einem Tor (5) angeordnet sind, das dauerhaft an dem Basisteil (1) angebracht und in Bezug auf das Basisteil (1) schwenkbar ist, **dadurch gekennzeichnet, dass** das Tor (5), nachdem die Kanüle (4) eingeführt und die Einführungsnadel entfernt worden ist, mindestens eine Position aufweist, in der ein Winkel α zwischen einer Längsmittelachse (I₂) des Tors (5) und der Längsmittelachse der Kanüle (4) besteht und dass der Winkel α nicht 0° und/oder 180° beträgt.

2. Vorrichtung nach Anspruch 1, **dadurch gekennzeichnet, dass** die Kanüle (4) oder eine flussigkeitsdichte Fortsetzung der Kanüle (4) flexibel ist.

3. Vorrichtung nach Anspruch 2, **dadurch gekennzeichnet, dass** die Kanüle (4) aus einem weichen Material hergestellt ist und nahe dem Austrittsende (7) des Tors (5) mit einem flexiblen Gelenk versehen ist.

4. Vorrichtung nach den Ansprüchen 1-3, **dadurch gekennzeichnet, dass** die Durchgangsöffnung mit Mitteln (30) versehen ist, die die Kanüle (4) vor dem Zugang von Mikroorganismen schützen.

5. Vorrichtung nach einem der Ansprüche 1-4, **dadurch gekennzeichnet, dass** die Durchgangsöffnung des Tores (5) und die Durchgangsöffnung der Kanüle (4) zusammenfallende Achsen (I2) haben.

6. Vorrichtung nach Anspruch 1, **dadurch gekennzeichnet, dass** das Tor (5) rohrförmig ist und dass das Tor (5) um eine Achse I₁ schwenken kann, wobei die Achse I₁ in einer Richtung senkrecht zu der Ebene angeordnet ist, die die Längsachse I₂ des Tors (5) umfasst.

7. Vorrichtung nach einem der Ansprüche 1-6, **dadurch gekennzeichnet, dass** die Achse I₁ unter der Außenfläche des Basisteils (1) in Verbindung mit dem Austrittsende (7) des Tores (5) angeordnet ist.

8. Vorrichtung nach Anspruch 7, **dadurch gekennzeichnet, dass** entweder das Tor (5) oder das Basisteil (1) mit rotationssymmetrischen, vorstehenden Teilen (10) versehen ist und der andere Teil mit entsprechenden Hohlräumen oder Öffnungen (11) versehen ist, die an der Rotationsachse I₁ des Tores (5) positioniert sind.

9. Vorrichtung nach den Ansprüchen 6-8, **dadurch gekennzeichnet, dass** das Tor (5) in Bezug auf das Basisteil (1) unlösbar montiert ist.

10. Kit umfassend eine Vorrichtung nach den Ansprüchen 1-9 und eine Injektionsvorrichtung (20), wobei die Zugangsöffnung (6) und die Kanüle (4) des Basisteils (1) so angeordnet sind, dass eine Injektionsnadel (9) auf beide ausgerichtet werden kann.

11. Kit nach Anspruch 10, **dadurch gekennzeichnet, dass** die Injektionsvorrichtung (20) in eine geschützte Position gebracht werden kann, in der die Injektionsnadel (9) innerhalb des Basisteils (1) positioniert ist.

12. Kit nach Anspruch 11, **dadurch gekennzeichnet, dass** das Montagekissen (2) mit einer Öffnung (2a) versehen ist, durch die die Injektionsnadel (9) hindurchgeführt werden kann.

13. Kit nach den Ansprüchen 10-12, **dadurch gekennzeichnet, dass** die Injektionsvorrichtung (20) in einem beliebigen Winkel ≤ 90 Grad in Bezug auf die untere/distale Oberfläche des Basisteils (1) angeordnet werden kann.

14. Kit nach den Ansprüchen 10-13, **dadurch gekennzeichnet, dass** das Kit dem Anwender in einer Verpackung unter sterilisierten Bedingungen geliefert wird.

## Revendications

1. Dispositif pour fournir du médicament comportant une partie (1) de base ayant des moyens (2) pour fixer la partie (1) de base à la peau d'un patient, une canule (4) reliée de manière non libérable à la partie (1) de base et s'étendant au moins partiellement à partir du côté proximal de la partie (1) de base et une ouverture de traversée fournissant l'accès de fluide à la canule (4), l'ouverture de traversée ayant une ouverture (6) d'accès et une ouverture (7) de sortie, l'ouverture (6) d'accès et au moins une partie de l'ouverture de traversée étant disposées dans une grille (5) fixée de manière permanente à la partie (1) de base et pouvant pivoter par rapport à la partie (1) de base, **caractérisé en ce que** la grille (5), après que la canule (4) a été insérée et que l'aiguille d'insertion a été retirée, a au moins une position dans laquelle il est formé un angle α entre un axe (I₂) longitudinal de la grille (5) et l'axe central longitudinal de la canule (4) et **en ce que** l'angle α est différent de 0° et/ou de 180°.

2. Dispositif suivant la revendication 1, **caractérisé en ce que** la canule (4) ou une continuation étanche au fluide de la canule (4) est souple.

3. Dispositif suivant la revendication 2, **caractérisé en ce que** la canule (4) est construite en un matériau mou et est munie d'un joint souple à proximité de l'extrémité (7) de sortie de la grille (5).

4. Dispositif suivant les revendications 1 à 3, **caractérisé en ce que** l'ouverture de traversée est munie de moyens (30) protégeant la canule (4) de l'accès de microorganismes.

5. Dispositif suivant l'une quelconque des revendications 1 à 4, **caractérisé en ce que** l'ouverture de traversée de la grille (5) et l'ouverture de traversée de la canule (4) ont des axes (I2) coïncidents.

6. Dispositif suivant la revendication 1, **caractérisé en ce que** la grille (5) est tubulaire et **en ce que** la grille (5) peut pivotée autour d'un axe (I₁), axe (I₁) qui est positionné dans une direction perpendiculaire au plan comportant l'axe I₂ longitudinal de la grille (5).

7. Dispositif suivant l'une quelconque des revendications 1 à 6, **caractérisé en ce que** l'axe I₁ est positionné en dessous de la surface extérieure de la partie (1) de base en connexion avec l'extrémité (7) de sortie de la grille (5).

8. Dispositif suivant la revendication 7, **caractérisé en ce que** soit la grille (5), soit la partie (1) de base est munie de parties (10) en saillie symétriques de révolution et l'autre partie est munie d'ouvertures (11) ou cavités correspondantes positionnées à l'axe I₁ de rotation de la grille (5).

9. Dispositif suivant les revendications 6 à 8, **caractérisé en ce que** la grille (5) est montée de manière non libérable par rapport à la partie (1) de base.

10. Kit comportant un dispositif suivant les revendications 1 à 9 et un dispositif (20) d'injection, l'ouverture (6) d'accès et la canule (4) de la partie (1) de base étant placées de sorte qu'une aiguille (9) d'injection peut être alignée avec toutes les deux.

11. Kit suivant la revendication 10, **caractérisé en ce que** le dispositif (20) d'injection peut être placé dans une position protégée où l'aiguille (9) d'insertion est positionnée à l'intérieur de la partie (1) de base.

12. Kit suivant la revendication 11, **caractérisé en ce que** le patin (2) de montage est muni d'une ouverture (2a) par laquelle ouverture peut passer l'aiguille (9) d'injection.

13. Kit suivant les revendications 10 à 12, **caractérisé en ce que** le dispositif (20) d'injection peut être placé suivant un angle quelconque ≤ 90 degrés par rapport à la surface distale/inférieure de la partie (1) de base.

14. Kit suivant les revendications 10 à 13, **caractérisé en ce que** le kit est fourni à l'utilisateur dans un emballage dans des conditions stérilisées.
